# FASCICULE DE BREVET EUROPEEN

(11) **EP 0 970 689 B1**
(45) Date de publication et mention de la délivrance du brevet: **18.09.2002**
(21) Numéro de dépôt: 99401502.2
(22) Date de dépôt: 17.06.1999
(51) Int. Cl.: A61K 7/42, A61K 7/00, A61K 7/04

(54) **Produit de maquillage associant un pigment photochrome et un filtre U.V. ses utilisations**
Schminkprodukt mit einem photochromen Pigment und einem UV-Filter und seine Verwendung
Make-up product with a photochromic pigment and a UV-filter and its uses

(30) Priorité: 25.06.1998 FR 9808085
(43) Date de publication de la demande: 12.01.2000
(73) Titulaire: L'OREAL, 75008 Paris (FR)
(72) Inventeur: Simon, Jean-Christophe, 75012 Paris (FR)
(74) Mandataire: Lhoste, Catherine

(56) Documents cités:
- EP-A- 0 359 909
- EP-A- 0 526 712
- EP-A- 0 709 728
- EP-A- 0 847 751
- US-A- 5 730 961
- PATENT ABSTRACTS OF JAPAN vol. 5, no. 178 (C-078) & JP 56 100709 A (KANEBO), 12 août 1981 (1981-08-12)
- DATABASE WPI Week 9549 Derwent Publications Ltd., London, GB; AN 95-380265 XP002102228 "Photochromic complex for topical skin compsn. - comprises thin lamellar base and titanium oxide surface layer for excellent photochromic properties" & JP 07 258580 A (SHISEIDO), 9 octobre 1995 (1995-10-09)
- DATABASE WPI Week 9735 Derwent Publications Ltd., London, GB; AN 97-381408 XP002102229 "Glossy pigmet for photochromic baseplate - prepared by baking mixture of mica, titanium oxide and one or more metals or metal compounds" & JP 09 165532 A (SHISEIDO), 24 juin 1997 (1997-06-24)
- DATABASE WPI Week 9542 Derwent Publications Ltd., London, GB; AN 95-325371 XP002102248 "Photochromic composite for topical compsn. - has titania layer on surface of thin plate like base material, of specific conditional parameters" & JP 07 223816 A (SHISEIDO), 22 août 1995 (1995-08-22)

## Description

La présente invention se rapporte à un produit cosmétique destiné à un nouveau type de maquillage, associant un agent de coloration photochrome et un filtre de lumière ultraviolette (U.V). Ce produit comprend deux compositions cosmétiques de maquillage qui peuvent être appliquées successivement sur la peau aussi bien du visage que du corps humain, sur les lèvres et sur les phanères comme les ongles, les cils, les sourcils ou les cheveux. L'invention a aussi pour objet un procédé de maquillage bicouche.

Chaque composition peut être une poudre libre ou compactée, un fond de teint, un fard à joues ou à paupières, un produit anti-cerne, un blush, un rouge à lèvres, un crayon à lèvres ou à yeux, un vernis à ongles ou encore un produit de maquillage du corps.

Les compositions de maquillage connues sont constituées d'un véhicule approprié et de différents agents de coloration destinés à conférer une certaine couleur aux compositions, avant et/ou après leur application sur la peau, les lèvres ou les phanères.

La gamme d'agents de coloration actuellement utilisée par les cosméticiens est assez limitée ; ces agents sont principalement des pigments organiques, des laques, des pigments minéraux ou des pigments nacrés. Les laques permettent d'obtenir des couleurs vives, mais sont pour la plupart instables à la lumière, à la température et au pH. Certaines présentent également l'inconvénient de tacher la peau de manière disgracieuse après application, par dégorgement du colorant. Les pigments minéraux, en particulier les oxydes minéraux sont au contraire très stables, mais donnent des couleurs plutôt ternes et pâles. Les pigments nacrés quant à eux permettent d'obtenir des couleurs variées, mais jamais intenses, à effets irisés mais le plus souvent assez faibles.

Par ailleurs, certains produits de maquillage classiques permettent de créer des effets de décoration avec des motifs colorés : dessins, damiers, lettres etc. Cependant, ces motifs sont visibles quelque soit la nature de la lumière, ce qui rend le maquillage « statique ».

Les maquilleurs et les consommateurs recherchant de plus en plus des effets spéciaux et des couleurs originales, le demandeur a découvert un nouveau type de maquillage utilisant des agents photochromes. En utilisant un produit bicouche dont la sous-couche contient au moins un agent de coloration photochrome, le demandeur a trouvé de façon surprenante qu'il était possible de tracer ou dessiner des motifs sur une telle couche (lettres, dessins, damiers...), en particulier avec un crayon ou pinceau, et que selon la nature de la lumière, les motifs apparaissaient ou disparaissaient L'invention permet un nouvel effet de maquillage « ludique » : les motifs colorés ou non apparaissent et disparaissent selon l'éclairage de la personne maquillée, comme "l'encre sympathique". Le maquillage semble ainsi « vivant ».

Un agent de coloration photochrome est un agent ayant la propriété de changer de couleur lorsqu'il est éclairé par de la lumière ultraviolette et de reprendre sa couleur initiale lorsqu'il n'est plus éclairé par cette lumière ou encore de passer d'un état non coloré à un état coloré et inversement. En particulier, cet agent présente des couleurs différentes selon qu'il est éclairé par de la lumière naturelle ou de la lumière artificielle.

Ce type d'agent est décrit dans le document US-5,730,961 qui propose un vernis à ongles contenant un composé photochrome capable de changer de couleur en présence de lumière ultraviolette. Cependant, ce document n'évoque nullement la possibilité d'avoir un produit bicouche, l'une des couches contenant un agent photochrome, et l'autre couche contenant un filtre UV, ni la possibilité de former des motifs qui apparaissent ou disparaissent selon la nature de la lumière, comme décrit ci-dessus.

De façon plus précise, l'invention a pour objet un produit cosmétique de maquillage comprenant des première et seconde compositions contenant un milieu cosmétiquement acceptable, conditionnées séparément, la première composition contenant au moins un agent de coloration photochrome susceptible de produire au moins une couleur en présence de lumière ultraviolette et la seconde composition contenant au moins un filtre de lumière ultraviolette.

L'invention a encore pour objet un procédé de maquillage de la peau, des lèvres et/ou des phanères d'être humain, consistant à appliquer sur la peau, les lèvres et/ou les phanères une première couche d'une première composition comprenant un milieu cosmétiquement acceptable et au moins un agent de coloration photochrome susceptible de produire au moins une couleur en présence de lumière ultraviolette puis à appliquer sur une partie de ladite première couche une seconde couche d'une seconde composition comprenant un milieu cosmétiquement acceptable et au moins un filtre de lumière ultraviolette.

Le produit de maquillage est notamment un kit de maquillage dans lequel ces deux compositions sont conditionnées dans des compartiments ou récipients distincts et sont accompagnés de moyens d'application appropriés.

L'architecture bicouche selon l'invention peut être adaptée pour tous les produits de maquillage de la peau aussi bien du visage que du scalp et du corps humain, des muqueuses comme les lèvres et l'intérieur des paupières inférieures, des phanères comme les ongles, les cils les cheveux, les sourcils, voire les poils. La seconde couche qui forme des motifs, peut être appliquée avec un stylo, crayon ou tout autre instrument (éponge, doigt, pinceau, brosse, plume etc.). Cette architecture bicouche peut aussi être appliquée sur les accessoires de maquillage comme les faux ongles, faux cils, perruques ou encore des pastilles ou des patchs adhérents sur la peau ou les lèvres (du type mouches).

L'invention se rapporte aussi à un support maquillé comprenant une première couche d'une première composition comportant au moins un agent de coloration photochrome et une seconde couche d'une seconde composition déposée en partie sur la première couche et comportant au moins un filtre de lumière ultraviolette.

La première couche formant une couche de base est aussi appelée couche photochrome et la couche de surface (top coat en terminologie anglo-saxonne) est aussi appelée couche filtrante.

Selon l'invention, la seconde couche est appliquée sur une partie seulement de la première couche. Elle peut être appliquée soit à l'une des extrémités de la première couche, soit au milieu, ou encore de façon discontinue notamment sous forme de motifs géométriques, symétriques ou dissymétriques (par exemple sous forme de points, carrés, ronds, étoiles, inscriptions alphanumériques ou tout autre symbole figuratif ou non), répartis de façon aléatoire ou ordonnée, à contours précis ou flous. Ainsi, les parties de la couche de base recouvertes de motifs sont protégées de la lumière ultraviolette et ne peuvent pas être excitées par cette dernière à l'inverse des parties de la couche de base, exposées.

Selon la nature de l'agent de coloration photochrome, et en présence ou non de lumière ultraviolette, les motifs de la couche de surface apparaîtront sur un fond coloré (couleur du photochrome excité) ou bien disparaîtront.

Les agents de coloration photochrome peuvent être quelconques. Lorsque la composition photochrome est transparente en l'absence de lumière ultraviolette et colorée en présence de lumière ultraviolette, les motifs n'apparaissent pas lorsque le support maquillé n'est pas éclairé en lumière ultraviolette (par exemple à l'intérieur d'une habitation) alors qu'ils apparaissent lorsque le support est éclairé en lumière ultraviolette (par exemple par la lumière du jour à l'extérieur des habitations ou sous des lampes ultraviolettes), par différence de couleur entre le fond et les motifs. Lorsque les motifs apparaissent, ils ont en particulier la couleur du support et notamment la couleur de la peau, des lèvres, des cils, des ongles ou des cheveux, alors que le fond est de la couleur de l'agent photochrome excité.

Lorsque la composition photochrome est colorée en l'absence de lumière ultraviolette et transparente en présence de lumière ultraviolette, les motifs apparaissent colorés lorsque le support maquillé n'est pas éclairé par de la lumière ultraviolette alors qu'ils n'apparaissent pas lorsque le support maquillé est éclairé par de la lumière ultraviolette.

Lorsque la composition photochrome est colorée en une première couleur en l'absence de lumière ultraviolette et colorée en une seconde couleur en présence de lumière ultraviolette, les motifs (de la couleur du support) apparaissent sur un fond coloré de la première couleur en l'absence de lumière ultraviolette et ils apparaissent sur un fond coloré de la seconde couleur en présence de lumière ultraviolette.

Afin de faire apparaître des motifs de couleur sur un fond coloré en une autre couleur, il est possible d'adjoindre à la composition contenant le filtre au moins un agent de coloration monocolore comme ceux classiquement utilisés en cosmétique.

L'invention a aussi pour objet, l'utilisation du produit ci-dessus pour faire apparaître ou disparaître sur la peau et/ou les lèvres et/ou les phanères d'êtres humains des motifs de couleur selon la présence ou non de la lumière ultra-violette.

La première composition de l'invention peut comprendre un ou plusieurs agents de coloration photochromes. De préférence, on utilise un seul agent photochrome pour une facilité de mise en oeuvre et un coût de fabrication réduit. Ces agents photochromes peuvent être des pigments ou des colorants.

Les agents photochromes utilisables dans l'invention sont en particulier ceux décrits dans les documents DE-A-196 43 773, JP-A-08/0209119, JP-A-09/100469, JP-A-09/183969, JP-A-09/095670, JP-A-09/031453, JP-A-09/165532, JP-A-08/217985, WO-A-95/20184, JP-A-07/258580, JP-A-07/223816, WO-A-89/12084, EP-A-624553, JP-A-08/337422, JP-A-07/145371, JP-A-63/308014, JP-A-07/0256617, EP-A-359909, FR-A-1 604 929, DE-A-19643773, US-A-5730961.

Plus précisément, les agents coloration photochromes utilisables dans l'invention sont les spiro-oxazines et leurs dérivés comme les spiroindolino-naphto-oxazines, les spironaphtoxazines, le naphtopyrane et ses dérivés, les spiropyrannes, comme les indolinospirobenzopyrannes, les nitrobenzylpyridines, les spirolanes, les oxydes de titane ou de zinc dopé par du fer. A titre d'exemple, on peut citer les agents photochromes du type dérivés de naphtopyrane vendus par la société PPG sous les références Photosol 5-68 Photochromic Dye, Photosol 7-49 Photochromic Dye, Photosol 7-106 Photochromic Dye, Photosol 0265 Photochromic Dye, Photosol 0272 Photochromic Dye, ces agents présentant deux couleurs différentes selon qu'ils sont excités ou non par des U.V. On peut aussi utiliser des aluminosilicates dopés notamment par les groupements S, Se, SO₄²⁻, WO₄²⁻, ou OH ou encore par des ions métalliques notamment de Fe, Cr, Mn, Co, Ni.

La seconde composition selon l'invention peut contenir un ou plusieurs filtres de lumière ultraviolette. Ces filtres peuvent être des filtres inorganiques comme les poudres d'oxyde de titane ou de zinc, notamment de taille nanométrique, ou encore des filtres organiques. Ces filtres peuvent être hydrophiles ou lipophiles et être actifs dans l'UVA et/ou l'UVB.

Les filtres organiques peuvent être notamment choisis parmi les dérivés cinnamiques, les dérivés salicyliques (filtres lipophiles), les dérivés du camphre, les dérivés sulfoniques de benzimidazole, les dérivés de triazine (filtres lipophiles), les dérivés de la benzophénone, les dérivés du dibenzoylméthane, les dérivés de β,β-diphénylacrylate, les dérivés de l'acide p-aminobenzoïque (filtres hydrophiles), les polymères filtres et silicones filtres lipophiles, décrits dans la demande WO-93/04665 et leurs mélanges.

### a) Les filtres hydrophiles

Comme filtres hydrophiles utilisables dans l'invention, on peut citer ceux décrits dans la demande EP-A-678292. Ces filtres comportent au moins un radical acide carboxylique ou sulfonique.

Comme exemple de filtres à groupe SO₃H, on peut citer les dérivés sulfoniques du 3-benzylidène 2-camphre et notamment ceux de formules (I), (II), (III), (IV), et (V) suivantes : 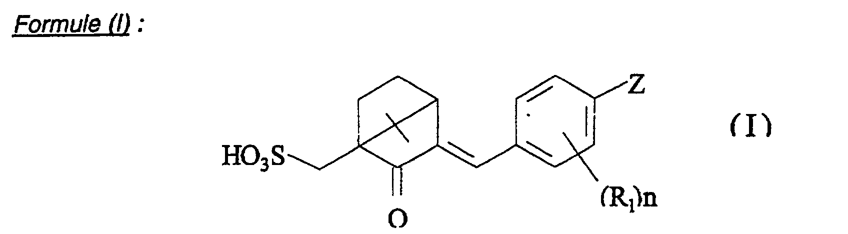 dans laquelle :
- Z désigne un groupement 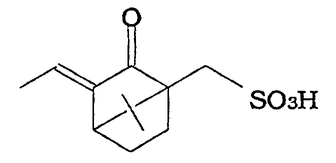
- n est égal à 0 ou est un nombre entier allant de 1 à 4.
- R₁ représente un ou plusieurs radicaux alkyle ou alkoxy, identiques ou différents, linéaires ou ramifiés, contenant de 1 à 4 atomes de carbone.

En particulier, on utilise l'acide benzène 1,4 [di(3-méthylidènecampho 10-sulfonique)] (n = 0 dans la formule I), filtre connu sous le nom commercial de *Mexoryl SX.* 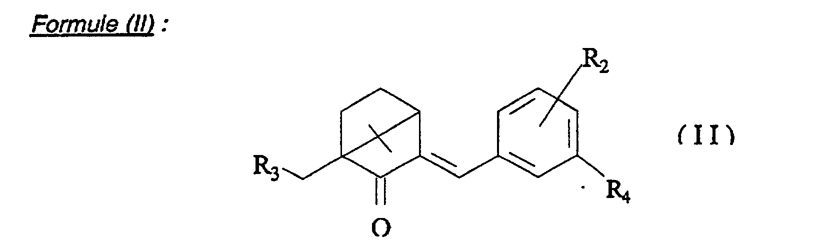 dans laquelle :
- R₂ désigne un atome d'hydrogène ou d'halogène, un radical alkyle ayant de 1 à 4 atomes de carbone ou un radical -SO₃H,
- R₃ et R₄ désignent un atome d'hydrogène ou un radical -SO₃H, l'un au moins des radicaux R₂, R₃ ou R₄ désignant le radical -SO₃H, R₂ et R₄ ne désignant pas simultanément un radical -SO₃H.

On peut citer, comme exemples particuliers de composés de formule (Il), l'acide 4-(3-méthylidènecamphre) benzène sulfonique (R₂ =-SO₃H en position para du benzylidènecamphre et R₃ et R₄ =H) ; l'acide 3-benzylidène campho-10-sulfonique (R₂ et R₄ = H et R₃ =-SO₃H) ; l'acide 2-méthyl 5-(3-méthylidènecamphre) benzène sulfonique (R₂ = méthyle en position para du benzylidène-camphre, R₄ = -SO₃H et R₃ = H) ; l'acide 2-chloro 5-(3-méthylidènecamphre) benzène sulfonique (R₂ = chlore en position para du benzylidènecamphre, R₄ = -SO₃H et R₃ = H) ; l'acide 3-(4-méthyl) benzylidène campho 10-sulfonique (R₂ = méthyle en position para du benzylidènecamphre, R₄ = H et R₃= -SO₃H). 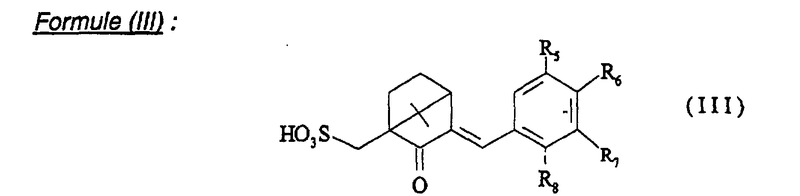 dans laquelle :
- R₅ et R₇ désignent un atome d'hydrogène, un radical hydroxyle, un radical alkyle ou alcoxy, linéaire ou ramifié, ayant de 1 à 8 atomes de carbone, l'un au moins des radicaux R₅ et R₇ représentant un radical hydroxyle, alkyle ou alcoxy,
- R₆ et R₈ désignent un atome d'hydrogène, un radical hydroxyle, l'un au moins des radicaux R₆ et R₈ désignant le radical hydroxyle, sous réserve que lorsque R₅ et R₈ désignent un atome d'hydrogène et que R₆ désigne un radical hydroxyle, R₇ ne désigne pas un radical alcoxy ou un atome d'hydrogène.

On peut citer, comme exemples particuliers de composés de formule (III), l'acide (3-t-butyl 2-hydroxy 5-méthyl) benzylidène campho-10-sulfonique (R₅ = CH₃, R₆ = H, R₇ = tertio-butyle, R₈ = -OH) ; l'acide (3-t-butyl 2-hydroxy 5-méthoxy) benzylidène campho-10-sulfonique (R₅ = méthoxy, R₆ = H, R₇ = tertio-butyle, R₈ = hydroxyle) ; l'acide (3,5-diterbutyl 4-hydroxy) benzylidène campho-10-sulfonique (R₅ = R₇ = tertiobutyle, R₆ = hydroxyle, R₈ = H). 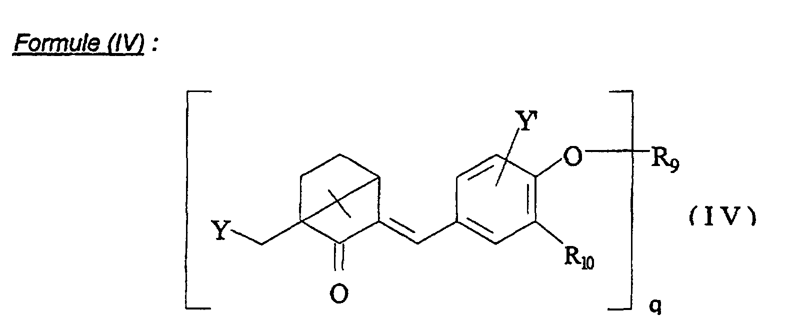 dans laquelle :
- R₉ désigne un atome d'hydrogène, un radical alkyle, linéaire ou ramifié, ayant de 1 à 18 atomes de carbone, un radical alcényle, linéaire ou ramifié, ayant de 3 à 18 atomes de carbone, un groupement 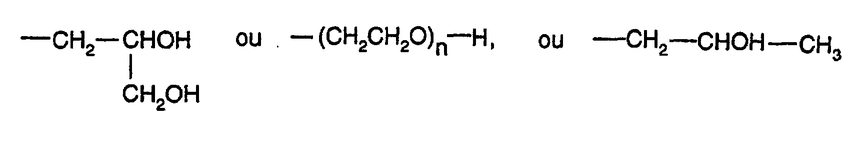
ou encore un radical divalent : -(CH₂)ₘ- ou -CH₂ -CHOH-CH₂-, n étant un nombre entier allant de 1 à 6 et m un nombre entier allant de 1 à 10
- R₁₀ désigne un atome d'hydrogène, un radical alcoxy ayant de 1 à 4 atomes de carbone ou un radical divalent - O - relié au radical R₉ lorsque celui-ci est aussi divalent,
- q désigne un nombre entier égal à 1 ou 2, étant entendu que si q est égal à 2, R₉ doit désigner un radical divalent,
- Y et Y' désignent un atome d'hydrogène ou un radical -SO₃H, au moins un de ces radicaux Y ou Y' étant différent de l'hydrogène.

On peut citer, comme exemples particuliers de composés de formule (IV), l'acide 2-méthoxy 5-(3-méthylidènecamphre) benzène sulfonique (q = 1, Y = R₁₀ = H, R₉ = méthyle, Y' en position 3 = -SO₃H) ; l'acide 3-(4,5-méthylènedioxy) benzylidène campho-10-sulfonique (q = 1, Y = -SO₃H, Y' = H, R₁₀ = -O- lié à R₉ désignant un radical méthylène) ; l'acide 3-(4-méthoxy) benzylidène campho-10-sulfonique(q = 1, Y = -SO₃H, Y' = R₁₀ =H, R₉ = CH₃) ; l'acide 3-(4,5-diméthoxy) benzylidène campho-10-sulfonique (q = 1, Y = -SO₃H, Y' = H, R₉ = méthyle, R₁₀ = méthoxy) ; l'acide 3-(4-n-butoxy) benzylidène campho-10-sulfonique (q = 1, ; Y = -SO₃H, Y' = R₁₀ = H, et R₉ = n-butyle) ; l'acide 3-(4-n-butoxy 5-méthoxy) benzylidène campho-10-sulfonique (q = 1, Y = -SO₃H, Y' = H, R₉ = n-butyle, R₁₀ = méthoxy). 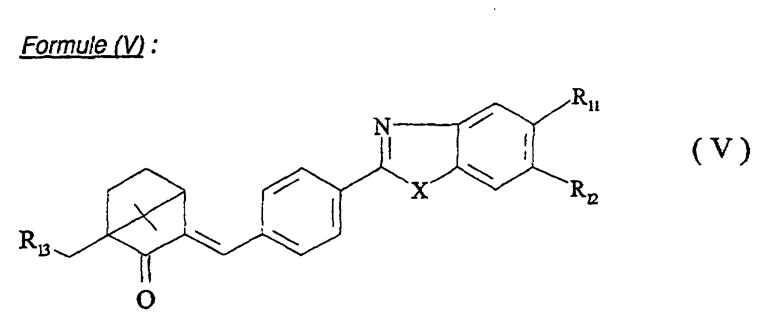 dans laquelle :
- R₁₁ désigne un atome d'hydrogène, un radical alkyle ou alcoxy, linéaire ou ramifié, ayant de 1 à 6 atomes de carbone ou un radical -SO₃H,
- R₁₂ désigne un atome d'hydrogène, un radical alkyle ou alcoxy, linéaire ou ramifié, ayant de 1 à 6 atomes de carbone,
- R₁₃ désigne un atome d'hydrogène ou un radical -SO₃H, l'un au moins des radicaux R₁₁ et R₁₃ désignant un radical -SO₃H,
- X est un atome d'oxygène ou de soufre ou un groupement -NR-, R étant un atome d'hydrogène ou un radical alkyle, linéaire ou ramifié, ayant de 1 à 6 atomes de carbone.

On peut citer, comme exemple particulier de composé de formule (V), l'acide 2-[4-(camphométhylidène) phényl] benzimidazole-5-sulfonique avec X = -NH-, R₁₁ = -SO₃H, R₁₂=R₁₃=H.

Les composés de formules (I), (Il), (III), (IV), (V) sont respectivement décrits dans les documents US-A-4 585 597, FR-A-2 236 515, FR-A-2 282 426, FR-A-2 645 148, FR-A-2 430 938 et FR-A-2 592 380.

Le filtre peut également être un dérivé sulfonique de la benzophénone de formule (VI) : 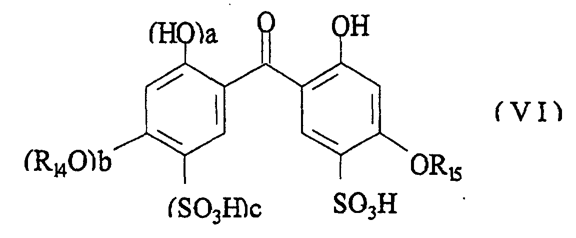 dans laquelle :
- R₁₄ et R₁₅, identiques ou différents, désignent soit un atome d'hydrogène soit un radical alkyle, linéaire ou ramifié, ayant de 1 à 8 atomes de carbone,
- a, b et c, identiques ou différents, sont des nombres égaux à 0 ou 1.

On peut citer comme exemple particulier de composé de formule (VI) : l'acide 2-hydroxy 4-méthoxybenzophénone 5-sulfonique (avec a, b, et c égaux à zéro, et R₁₅ = méthyle).

Le filtre peut encore être un dérivé sulfonique de formule (VII) : 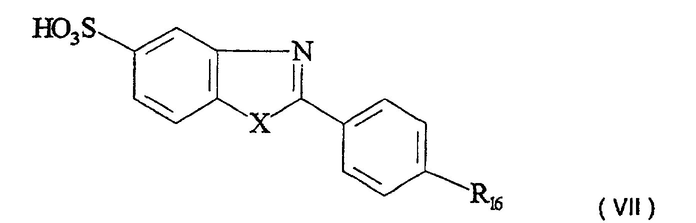 dans laquelle :
- X désigne un atome d'oxygène ou un radical -NH-,
- R₁₆ désigne un atome d'hydrogène, un radical alkyle ou alcoxy, linéaire ou ramifié, ayant de 1 à 8 atomes de carbone ou un groupement de formule (VIII) 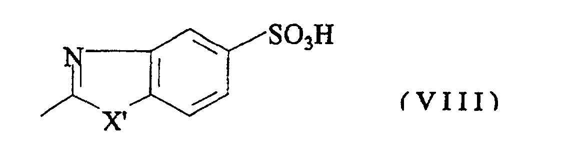 dans laquelle X' représente un atome d'oxygène ou un radical -NH-.

On peut citer, comme exemple particulier de composé de formule (VII), l'acide 2-phényl-benzimidazole 5-sulfonique avec X = -NH- et R₁₆ = H. Ce filtre de rayonnements UV-B est vendu sous le nom « EUSOLEX 232 » par la société MERCK. On peut aussi utiliser, l'acide benzène 1,4 -di(benzimidazol -2 yl-5-sulfonique) avec X = -NH-, R₁₆ désignant le groupement de formule (VIII) dans lequel X' = -NH- ; l'acide benzène 1,4-di (benzoxazol -2 yl -5-sulfonique) avec X = O, R₁₆ désignant le groupement de formule (VIII) dans lequel X' = O.

### b) Les filtres lipophiles

Comme filtres lipophiles utilisables dans l'invention, on peut citer les dérivés du dibenzoylméthane et plus spécialement le 4-tert.-butyl-4'-méthoxy dibenzoylméthane. Ces dérivés du dibenzoylméthane, qui sont des filtres actifs dans l'UV-A, sont notamment décrits dans les documents FR-A-2 326 405, FR-A-2 440 933, et EP-A- 0 114 607 ; le 4-(ter.-butyl) 4'-méthoxy dibenzoylméthane est vendu sous le nom de "PARSOL 1789" par la Société GIVAUDAN. Il présente la formule développée suivante :

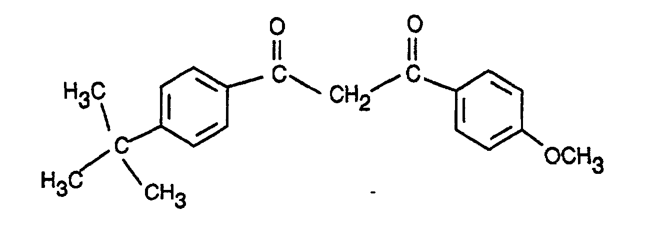

Un autre dérivé du dibenzoylméthane utilisable dans l'invention est le 4-isopropyldibenzoylméthane, filtre vendu sous la dénomination de "EUSOLEX 8020" par la Société MERCK, et répondant à la formule développée suivante :

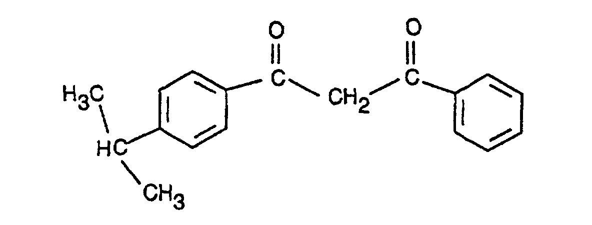

On peut aussi utiliser, l'a-cyano-b, b-diphénylacrylate de 2-éthylhéxyle, encore appelé octocrylène, filtre lipophile liquide ayant une activité dans l'UV-B, disponible commercialement sous la dénomination de "UVINUL N 539" par la Société BASF. Il répond à la formule suivante : 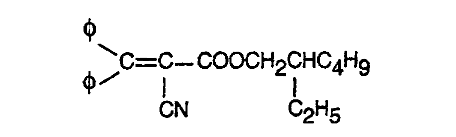 dans laquelle Φ désigne un radical phényle.

Comme autre filtre lipophile utilisable dans l'invention on peut encore citer le p-méthylbenzylidène camphre connu comme absorbant dans l'UV-B et vendu notamment sous la dénomination commerciale « EUSOLEX 6300 » par la société Merck.

Les nanopigments d'oxyde de titane ou de zinc utilisables comme filtre dans les compositions selon l'invention peuvent être traités en surface ou non traités. L'oxyde de titane peut se présenter sous forme rutile, anatase ou amorphe, mais de préférence sous forme rutile et/ou anatase.

Par nanopigments, on entend des pigments dont la taille moyenne des particules élémentaires est comprise entre 5 et 100 nm.

Les nanopigments traités peuvent par exemple être traités par l'alumine, la silice, les composés de l'aluminium, les composés du silicium, les composés du sodium, les oxydes de fer, les esters de fer, l'acide stéarique, la glycérine.

Plus particulièrement, les nanopigments traités peuvent être des oxydes de titane traités par:
- la silice et l'alumine tels que les produits « MICROTITANIUM DIOXIDE MT 500 SA » et « MICROTITANIUM DIOXIDE MT 100 SA » de la société TAYCA, et les produits « TIOVEIL Fin », « TIOVEIL OP », TIOVEIL MOTG » et « TIOVEIL IPM » de la société TIOXIDE,
- l'alumine et le stéarate d'aluminium tels que le produit « MICROTITANIUM DIOXIDE MT 100 T » de la société TAYCA,
- l'alumine et le laurate d'aluminium tels que le produit « MICROTITANIUM DIOXIDE MT 100 S » de la société TAYCA,
- des oxydes de fer et le stéarate de fer tels que le produit « MICROTITANIUM DIOXIDE MT 100 F » de la société TAYCA,
- la silice, l'alumine et la silicone tels que les produits « MICROTITANIUM DIOXIDE MT 100 SAS », « MICROTITANIUM DIOXIDE MT 600 SAS » et « MICROTITANIUM DIOXIDE MT 500 SAS » de la société TAYCA,
- l'hexamétaphosphate de sodium tels que le produit « MICROTITANIUM DIOXIDE MT 150 W » de la société TAYCA,
- l'octyltriméthoxysilane tels que le produit « T-805 » de la société DEGUSSA,
- l'alumine et l'acide stéarique tels que le produit « UVT-M160 » de la société KEMIRA,
- l'alumine et la glycérine tels que le produit « UVT-M212 » de la société KEMIRA,
- l'alumine et la silicone tels que le produit « UVT-M262 » de la société KEMIRA.

Les nanooxydes de titane non traités peuvent par exemple être ceux vendus par la société TAYCA sous les dénominations commerciales « MICROTITANIUM DIOXIDE MT 500 B » ou « MICROTITANIUM DIOXIDE MT 600 B ».

Le ou les filtres peuvent être présents dans chaque composition selon l'invention à une teneur allant de 0,01 à 30 % du poids total de la composition, de préférence de 0,5 à 20 %. Selon la nature et la quantité de filtres utilisées, il est possible d'obtenir un filtrage des rayons U.V total ou partiel et d'obtenir différentes nuances d'intensité de couleur.

La seconde composition peut, en outre contenir des agents de coloration monocolores notamment choisis parmi les colorants monocolores, les pigments monocolores et les nacres classiquement utilisés dans les compositions cosmétiques, et leurs associations.

Par pigments, il faut comprendre des particules blanches ou colorées, minérales ou organiques, insolubles dans la phase grasse liquide, destinées à colorer et/ou opacifier la seconde composition. Par nacres, il faut comprendre des particules irisées, notamment produites par certains mollusques dans leur coquille ou bien synthétisées. Par colorants, il faut comprendre des composés généralement organiques solubles dans les corps gras comme les huiles ou dans une phase hydroalcoolique.

Les agents de coloration des première et seconde couches peuvent être présents à raison de 0,01 à 60 % du poids total respectivement de la première composition et seconde composition, de préférence de 0,05 à 30 % et plus particulièrement de 1 à 20 %, pour des compositions non pulvérulentes. Pour des compositions pulvérulentes, la quantité d'agents de coloration peut aller jusqu'à 85 % et même jusqu'à 98 %.

Comme pigments minéraux monocolores utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium.

Les colorants peuvent être liposolubles ou hydrosolubles. Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter de 0,01 à 20 % du poids total de la seconde composition et mieux de 0,1 à 10 %. Les colorants hydrosolubles sont notamment le sulfate de cuivre, de fer, des sulfopolyesters hydrosolubles tels que ceux décrits dans les documents FR-96 154152, les rhodamines, les colorants naturels (carotène, jus de betterave), bleu de méthylène. Les nacres peuvent être présentes dans la seconde composition à raison de 0 à 20 % du poids total de ladite seconde composition, de préférence à un taux de l'ordre de 1 à 15 %. Parmi les nacres utilisables dans la seconde composition, on peut citer le mica recouvert d'oxyde de titane, d'oxyde de fer, de pigment naturel ou d'oxychlorure de bismuth tel que le mica titane coloré.

Les première et seconde compositions selon l'invention peuvent, de plus, comprendre tous les ingrédients classiquement utilisés dans les domaines concernés et plus spécialement dans les domaines cosmétique et dermatologique. Ces ingrédients sont en particulier choisis parmi les corps gras, les conservateurs, les stabilisants, les neutralisants, les épaississants de phase aqueuse (biopolymères polysaccharidiques, les polymères synthétiques) ou de phase grasse comme les argiles, les charges, les parfums, les actifs hydrophiles ou lipophiles, les tensioactifs, les antioxydants, les polymères filmogènes et leurs mélanges. Les quantités de ces différents ingrédients sont celles classiquement utilisées dans les domaines concernés et par exemple de 0,01 % à 30 % du poids total de la composition. La nature de ces ingrédients et leur proportion doivent être compatibles avec l'obtention de compositions selon l'invention, stables épaissies et brillantes. La composition peut aussi contenir de l'eau à une concentration allant de 0 à 95% du poids total de la composition ou des solvants organiques pouvant représenter jusqu'à 90%.

Par charges, il faut comprendre des particules incolores ou blanches, minérales ou de synthèse, lamellaires ou non lamellaires. Ces charges peuvent être introduites dans les première ou seconde couches en vue de notamment modifier la texture -de ces compositions. Elles peuvent être présentes à raison de 0 à 35 % du poids total de la chaque composition, de préférence 0,5 à 15 %. On peut notamment citer le talc, le stéarate de zinc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Coming) et les microbilles de résine de silicone (Tospearl de Toshiba, par exemple) ou encore la silice.

Les première et seconde compositions de l'invention comprennent, avantageusement une phase grasse contenant des corps gras liquides, solides ou pâteux à température ambiante. Les corps gras solides à température ambiante permettent de structurer la composition ; ils sont choisis parmi les gommes et/ ou les cires. Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans les première et seconde compositions de l'invention, on peut citer, la lanoline, la cire d'abeilles, la cire de Carnauba ou de Candellila, la paraffine, les cires de lignite ou microcristalline, la. cérésine ou l'ozokérite ; les cires synthétiques comme les cires de polyéthylène, les cires de Fischer Tropsch, les cires de silicones comme les alkyl ou alkoxy-diméticone ayant de 16 à 45 atomes de carbone.

Les gommes sont notamment des organopolysiloxanes (du type PDMS) ayant un poids moléculaire moyen de 1000 à 500 000.

La nature et la quantité de ces gommes ou cires sont fonction des propriétés mécaniques et de textures recherchées. A titre indicatif, chaque composition peut contenir de 0 à 50 % en poids de cires, par rapport au poids total de la composition et mieux de 5 à 30 % et de 0 à 20 % de gomme.

Comme corps gras liquide à température, utilisable dans les compositions de l'invention, on peut citer :
- les huiles hydrocarbonées d'origine animale telles que le perhydrosqualène ;
- les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les isoparaffines comme l'isohexadécane et l'isodécane ;
- les esters et les éthers de synthèse notamment d'acides gras comme les huiles de formule R₁COOR₂ dans laquelle R₁ représente le reste d'un acide gras supérieur comportant de 7 à 29 atomes de carbone et R₂ représente une chaîne hydrocarbonée contenant de 3 à 30 atomes de carbone comme par exemple l'huile de Purcellin, le myristate d'isopropyle, le palmitate d'éthyl-2hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ;
- des alcools gras ayant de 12 à 26 atomes de carbone comme l'octyldodécanol, le 2-butyloctanol, le 2-hexyldécanol, le 2-undécylpentadécanol, l'alcool oléique ;
- les huiles fluorées partiellement hydrocarbonées et/ou siliconées comme celle décrites dans le document JP-A-2-295912 ;
- les huiles siliconées comme les polyméthylsiloxanes (PDMS) volatiles ou non, linéaires ou cycliques, liquides ou pâteux à température ambiante ; les silicones phénylées comme les phényl triméthicones, les diphényl diméthicones, les phényl diméthicones, les phényltriméthylsiloxy diphényl siloxanes ;
- les huiles fluorées et les huiles fluorosiliconées ;
- leurs mélanges.

Ces huiles peuvent représenter de 0 à 99,9% du poids total de chaque composition.

Comme huile de silicone volatile utilisable dans l'invention, on peut citer les silicones linéaires ou cycliques ayant de 2 à 7 atomes de silicium, ces silicones comportant éventuellement des groupes alkyle ayant de 1 à 10 atomes de carbone. Ainsi, ces silicones sont notamment l'hexaméthyldisiloxane, la cyclopenta- ou cyclotétra- ou cyclohexadiméthylsiloxane. Ces huiles volatiles peuvent représenter de 0 à 50% du poids total de la composition.

Comme solvant utilisable dans l'invention, on peut citer :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde.

Ces solvants conviennent plus particulièrement pour le maquillage et le soin des ongles : la composition constitue alors un vernis à ongles ou un produit de soin des ongles. Comme solvant on peut aussi utiliser l'eau et les milieux hydroalcooliques.

Comme polymère filmifiable utilisable dans l'invention, on peut citer la nitrocellulose, l'acétobutyrate de cellulose, les butyralpolyvinyliques, les résines alkydes, les résines résultant de la condensation de formaldéhyde avec une arylsulfonamide, les polyesters, les polyuréthannes, les polyester-polyuréthanes, les polyéther-polyuréthanes, les polymères radicalaires, notamment de type acrylique, acrylique styrène et/ou vinylique et leurs mélanges.

Les polymères peuvent être dissous ou dispersés dans la composition. Ils peuvent être présents à une teneur allant de 0 % à 40 % en poids par rapport au poids total de la composition, par exemple de 0,5 % à 40 % et mieux allant de 10 % à 20 % en poids.

La composition selon l'invention peut également comprendre, en plus du ou des polymères filmogènes, des agents plastifiants qui permettent de régler la flexibilité du film de polymère sans affaiblir sa résistance physique.

Les agents plastifiants utilisables sont ceux couramment employés dans les compositions de vernis à ongles. Comme plastifiants, on peut citer les phtalates de dibutyle, de dioctyle, de di-isobutyle, de diméthoxyéthyl, les benzoates de benzyle, de glycéryle ; les citrates de triéthyle, de tributyle, l'acétyl-citrate de tributyle ; les phosphates de tributyle, de triphényle ; les glycols le camphre ainsi que leurs dérivés et leurs mélanges. Les plastifiants peuvent être généralement présents à une teneur allant de 0 % à 30 % du poids total de la composition, par exemple de 1 à 30 % et mieux de 5 % à 10 %.

Les compositions de l'invention peuvent se présenter sous toutes les formes galéniques normalement utilisées pour une application topique, et notamment sous forme d'une solution huileuse ou aqueuse, d'un gel huileux ou aqueux, d'une émulsion huile-dans-eau ou eau-dans-huile, d'une dispersion d'huile dans de l'eau grâce à des vésicules, les vésicules étant situées à l'interface huile/eau ou encore d'une poudre. Chaque composition peut avoir l'aspect d'une lotion, d'une crème, d'une pommade, d'une pâte souple, d'un onguent, d'un solide coulé ou moulé et notamment en stick ou en coupelle, ou encore de solide compacté.

Le produit selon l'invention peut être avantageusement utilisé pour le maquillage de la peau et/ou des muqueuses et/ou des phanères selon la nature des ingrédients utilisés. En particulier, chaque composition de l'invention peut être sous forme de bâton ou pâte de rouge à lèvres, de fond de teint solide, de produit anti-cernes ou contours des yeux, d'eye liner, de mascara, d'ombre à paupières, de vernis à ongles à milieu solvant ou aqueux, de produit de maquillage du corps ou encore un produit de coloration de la peau. Ces compositions peuvent, en outre, contenir des actifs cosmétiques ou dermatologiques, en vue, notamment d'apporter un aspect soin ou traitant à la composition. Ainsi ces compositions peuvent contenir des vitamines et autres actifs lipophiles (lanoline) ou hydrophiles (hydratants comme la glycérine).

L'invention a encore pour objet un produit à lèvres, un fond de teint, un tatouage, un vernis à ongles, un fard à joues ou à paupières contenant un milieu cosmétiquement acceptable et des première et seconde compositions telles que décrites précédemment.
Les compositions de l'invention peuvent être obtenues par chauffage des différents constituants à la température de fusion des cires les plus élevées, puis coulage du mélange fondu dans un moule (coupelle ou doigt de gant). Elles peuvent aussi être obtenues par extrusion comme décrit dans la demande EP-A-667 146.

L'exemple de composition ci-après est donné à titre illustratif et sans caractère limitatif. Les quantités y sont données en % en poids.

### Exemple de réalisation : Vernis à ongle

**a)** La base de vernis (appelée base VAO) utilisée pour les deux couches est la suivante :
   - Hectorite modifiée 1,4%
   - Nitrocellulose (30 % dans alcool isopropylique) 14,5 %
   - Résine alkyde (30 %dans acétate d'éthyle) 16,5 %
   - Acétyl-citrate de tri-butyle 7,0 %
   - Alcool iso-propylique 3,7 %
   - Acétate d'éthyle 22,8 %
   - Acétate de butyle qsp 100 %
**b)** La composition pour former la couche de base de vernis à ongles est réalisée avec :
   - Base VAO 98,0 %
   - Photosol 5-3 Photochromic Dye de chez PPG 2,0 %

   Cette composition est obtenue en mélangeant à température ambiante la base VAO et le pigment photochrome sous agitation. Sa couleur change selon la présence ou non de rayons U.V. et varie de l'incolore au jaune. Cette composition est appliquée en continue sur des ongles démaquillés, sous forme de monocouche.
**c)** La composition pour former la couche de surface de vernis à ongles contient
   - Base VAO 95,0 %
   - Dioxyde de titane nanométrique (MT 100T) 5,0 %

La composition de la couche de surface est transparente. Elle est obtenue en mélangeant les pigments et la base VAO comme on le fait classiquement pour la fabrication d'un vernis.

Cette composition de surface est appliquée au pinceau sur la couche de base en formant des motifs (tâches, étoiles, papillons). Après séchage du vernis bicouche, ce dernier laisse apparaître l'ongle en l'absence de rayons U.V. (dans une habitation notamment). En revanche en présence de rayons U.V. (à la lumière du jour notamment), la couche de base devient jaune et fait ainsi apparaître des motifs de la couleur de l'ongle sur un fond jaune. Lorsque l'éclairement par des rayons U.V. disparaît, les motifs disparaissent.

## Revendications

1. Produit cosmétique de maquillage comprenant des première et seconde compositions contenant un milieu cosmétiquement acceptable, conditionnées séparément, la première composition contenant au moins un agent de coloration photochrome susceptible de produire au moins une couleur en présence de lumière ultraviolette et la seconde composition contenant au moins un filtre de lumière ultraviolette.

2. Produit selon la revendication 1, **caractérisé en ce que** l'agent de coloration photochrome est choisi parmi les spiro-oxazines et leurs dérivés, les spironaphtoxazines, le naphtopyrane et ses dérivés, les spiropyrannes, les nitrobenzylpyridines, les spirolanes, les oxydes de titane ou de zinc dopé par du fer, les aluminosilicates dopés par des ions métalliques ou par un groupement choisi parmi Se, S, OH, SO₄²⁻, WO₄²⁻.

3. Produit selon la revendication 1 ou 2, **caractérisé en ce que** le filtre de lumière ultraviolette est choisi parmi les nano-oxydes de titane ou de zinc, traités ou non, les dérivés cinnamiques, les dérivés salicyliques, les dérivés du camphre, les dérivés sulfoniques de benzimidazole, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres lipophiles, et leurs mélanges.

4. Produit selon l'une des revendications précédentes, **caractérisé en ce que** le filtre de lumière ultraviolette représente de 0,01 à 30 % et mieux de 0,5 à 20 % du poids total de la seconde composition.

5. Produit selon l'une des revendications précédentes, **caractérisé en ce que** la seconde composition contient au moins un agent de coloration monocolore.

6. Produit selon la revendication précédente, **caractérisé en ce que** l'agent de coloration monocolore est choisi parmi les colorants monocolores, les pigments monocolores et/ou les nacres.

7. Produit selon la revendication 5 ou 6, **caractérisé en ce que** l'agent de coloration monocolore est choisi parmi les oxydes de titane, de zirconium ou de cérium ainsi que les oxydes de zinc, de fer ou de chrome, le bleu ferrique, le noir de carbone, et les laques de baryum, de strontium, de calcium, ou d'aluminium, le rouge Soudan, le DC Red 17, le DC Green 6, le β-carotène, l'huile de soja, le brun Soudan, le DC Yellow, 11, le DC Violet 2, le DC orange 5, le jaune quinoléine, le mica recouvert d'oxyde de titane, d'oxyde de fer, de pigment naturel ou d'oxychlorure de bismuth tel que le mica titane coloré.

8. Produit selon l'une des revendications précédentes, **caractérisé en ce que** chaque agent de coloration représente de 0,01 à 98 % et mieux de 0,05 à 85 % du poids total respectivement de la première et seconde compositions.

9. Produit selon l'une des revendications précédentes, **caractérisé en ce qu'**il est conditionné sous forme de fond de teint, vernis à ongle, produit de maquillage du corps ou des lèvres, fard à joues ou à paupières.

10. Produit selon l'une des revendications précédentes, **caractérisé en ce que** chaque composition est sous forme d'une solution huileuse ou aqueuse, d'un gel huileux ou aqueux, d'une émulsion huile-dans-eau ou eau-dans-huile, d'une dispersion d'huile dans de l'eau grâce à des vésicules, les vésicules étant situées à l'interface huile/eau, ou d'une poudre.

11. Produit selon l'une des revendications précédentes, **caractérisé en ce que** le milieu cosmétiquement acceptable contient, en outre, au moins un ingrédient choisi parmi les huiles, les solvants, les cires, les polymères filmogènes, les charges, les actifs hydrophiles ou lipophiles, les épaississants de phase aqueuse ou grasse, les tensioactifs, les antioxydants, les parfums, les plastifiants, les neutralisants, les stabilisants, leurs conservateurs.

12. Procédé de maquillage de la peau, des lèvres et/ou des phanères d'être humain, consistant à appliquer sur la peau, les lèvres et/ou les phanères une première couche d'une première composition comprenant un milieu cosmétiquement acceptable et au moins un agent de coloration photochrome susceptible de produire au moins une couleur en présence de lumière ultra-violette, puis à appliquer sur une partie de ladite première couche une seconde couche d'une seconde composition comprenant un milieu cosmétiquement acceptable et au moins un filtre de lumière ultra-violette.

13. Procédé selon la revendication 12, **caractérisé en ce que** la seconde couche est discontinue.

14. Procédé selon l'une des revendications 12 à 13, **caractérisé en ce que** la seconde couche comprend des motifs.

15. Procédé selon l'une des revendications 12 à 14, **caractérisé en ce que** la seconde couche contient des motifs répartis de façon aléatoire ou ordonnée, de forme symétrique ou dissymétrique.

16. Procédé selon l'une des revendications 12 à 15, **caractérisé en ce que** l'agent de coloration photochrome est choisi parmi les spiro-oxazine et ses dérivés, les spironaphtoxazines, le naphtopyrane et ses dérivés, les spiropyrannes, les nitrobenzylpyridines, les spirolanes, les oxydes de titane ou de zinc dopé par du fer, les aluminosilicates dopés par des ions métalliques ou par un groupement choisi parmi Se, S, OH, SO₄²⁻, WO₄²⁻.

17. Procédé selon l'une des revendications 12 à 16, **caractérisé en ce que** la seconde composition contient au moins un agent de colorations additionnel monocolore.

18. Procédé selon l'une des revendications 12 à 17, **caractérisé en ce que** l'agent de coloration monocolore est choisi parmi les colorants monocolores, les pigments monocolores et/ou les nacres.

19. Procédé selon la revendication 17 à 18, **caractérisé en ce que** l'agent de coloration monocolore est choisi parmi les oxydes de titane, de zirconium ou de cérium ainsi que les oxydes de zinc, de fer ou de chrome, le bleu ferrique, le noir de carbone, et les laques de baryum, de strontium, de calcium, ou d'aluminium, le rouge Soudan, le DC Red 17, le DC Green 6, le β-carotène, l'huile de soja, le brun Soudan, le DC Yellow, 11, le DC Violet 2, le DC orange 5, le jaune quinoléine, le mica recouvert d'oxyde de titane, d'oxyde de fer, de pigment naturel ou d'oxychlorure de bismuth tel que le mica titane coloré.

20. Kit de maquillage comprenant un produit selon l'une des revendications 1 à 11.

21. Kit selon la revendication 20, **caractérisé en ce qu'**il contient des moyens pour appliquer sur la peau et/ou les muqueuses et/ou les phanères la première et la seconde compositions.

22. Kit selon la revendication 21, **caractérisé en ce que** les moyens sont choisis parmi les pinceaux, stylos, brosses, plumes, crayons.

23. Kit selon l'une des revendications 20 à 22, caractérisé en ce les première et seconde compositions sont conditionnées dans des compartiments ou récipients distincts.

24. Utilisation cosmétique du produit selon l'une des revendications 1 à 11 pour faire apparaître ou disparaître sur la peau et/ou les lèvres et/ou les phanères d'êtres humains des motifs de couleur selon la présence ou non de la lumière ultra-violette, par application sur la peau, les lèvres et/ou les phanères, d'une première couche de la première composition puis application, sur une partie de ladite première couche, d'une seconde couche de la seconde composition.

25. Support maquillé comprenant une première couche d'une première composition comportant au moins un agent de coloration photochrome susceptible de produire au moins une couleur en présence de lumière ultra-violette et une seconde couche d'une seconde composition déposée en partie sur la première couche et comportant au moins un filtre de lumière ultra-violette.

26. Support selon la revendication 25, **caractérisé en ce qu'**il se présente sous forme de faux ongles, faux cils, perruques.

27. Support selon la revendication 25 ou 26, caractérisé en ce la seconde couche est discontinue.

28. Support selon l'une des revendications 25 à 27, **caractérisé en ce que** la seconde couche comprend des motifs répartis de façon aléatoire ou ordonnée.

29. Support selon l'une des revendications 25 à 28, **caractérisé en ce que** l'agent de coloration photochrome est choisi parmi les spiro-oxazines et leurs dérivés, les spironaphtoxazines, le naphtopyrane et ses dérivés, les spiropyrannes, les nitrobenzylpyridines, les spirolanes, les oxydes de titane ou de zinc dopé par du fer, les aluminosilicates dopés par des ions métalliques ou par un groupement choisi parmi Se, S, OH, SO₄²⁻, WO₄²⁻.

30. Support selon l'une des revendications 25 à 29, **caractérisé en ce que** le filtre de lumière ultraviolette est choisi parmi les nano-oxydes de titane ou de zinc, traités ou non, les dérivés cinnamiques, les dérivés salicyliques, les dérivés du camphre, les dérivés sulfoniques de benzimidazole, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres lipophiles, et leurs mélanges.

31. Support selon l'une des revendications 25 à 30, **caractérisé en ce que** la seconde composition contient au moins un agent de coloration monocolore.

## Claims

1. Cosmetic make-up product comprising first and second compositions containing a cosmetically acceptable medium, which are packaged separately, the first composition containing at least one photochromic colouring agent capable of producing at least one colour in the presence of ultraviolet light, and the second composition containing at least one UV light screening agent.

2. Product according to Claim 1, **characterized in that** the photochromic colouring agent is chosen from spirooxazines and derivatives thereof, spironaphthoxazines, naphthopyran and derivatives thereof, spiropyrans, nitrobenzylpyridines, spirolanes, titanium or zinc oxides doped with iron, aluminosilicates doped with metal ions or with a group chosen from Se, S, OH, SO₄²⁻ and WO₄²⁻.

3. Product according to Claim 1 or 2, **characterized in that** the UV light screening agent is chosen from treated or untreated titanium or zinc nanooxides, cinnamic derivatives, salicylic derivatives, camphor derivatives, sulphonic benzimidazple derivatives, triazine derivatives, benzophenone derivatives, dibenzoylmethane derivatives, β,β-diphenylacrylate derivatives, p-aminobenzoic acid derivatives, lipophilic screening polymers and screening silicones, and mixtures thereof.

4. Product according to one of the preceding claims, **characterized in that** the UV light screening agent represents from 0.01 to 30% and better still from 0.5 to 20% relative to the total weight of the second composition.

5. Product according to one of the preceding claims, **characterized in that** the second composition contains at least one monochromatic colouring agent.

6. Product according to the preceding claim, **characterized in that** the monochromatic colouring agent is chosen from monochromatic dyes, monochromatic pigments and/or pearlescent agents.

7. Product according to Claim 5 or 6, **characterized in that** the monochromatic colouring agent is chosen from titanium oxide, zirconium oxide or cerium .oxide as well as zinc oxide, iron oxide or chromium oxide, ferric blue, carbon black and barium, strontium, calcium or aluminium lakes, Sudan red, DC Red 17, DC Green 6, β-carotene, soybean oil, Sudan brown, DC Yellow 11, DC Violet 2, DC Orange 5, quinoline yellow, mica coated with titanium oxide, with iron oxide, with natural pigment or with bismuth oxychloride, such as coloured titanium mica.

8. Product according to one of the preceding claims, **characterized in that** each colouring agent represents from 0.01 to 98% and better still from 0.05 to 85% relative to the total weight respectively of the first and second compositions.

9. Product according to one of the preceding claims, **characterized in that** it is packaged in the form of a foundation, a nail varnish or a make-up product for the body or the lips, face powder or an eye shadow.

10. Product according to one of the preceding claims, **characterized in that** each composition is in the form of an oily or aqueous solution, an oily or aqueous gel, an oil-in-water or water-in-oil emulsion, a dispersion of oil in water by means of vesicles, the vesicles being at the oil/water interface, or a powder.

11. Product according to one of the preceding claims, **characterized in that** the cosmetically acceptable medium also contains at least one ingredient chosen from oils, solvents, waxes, film-forming polymers, fillers, hydrophilic or lipophilic active agents, aqueous-phase thickeners or fatty-phase thickeners, surfactants, antioxidants, fragrances, plasticizers, neutralizing agents, stabilizers and preserving agents.

12. Make-up process for human skin, lips and/or superficial body growths, which consists in applying a first coat of a first composition comprising a cosmetically acceptable medium and at least one photochromic colouring agent capable of producing at least one colour in the presence of ultraviolet light to the skin, the lips and/or superficial body growths, and then in applying a second coat of a second composition comprising a cosmetically acceptable medium and at least one UV light screening agent, over a part of the said first coat.

13. Process according to Claim 12, **characterized in that** the second coat is discontinuous.

14. Process according to either of Claims 12 and 13, **characterized in that** the second coat comprises patterns.

15. Process according to one of Claims 12 to 14, **characterized in that** the second coat contains patterns distributed in a random or ordered manner, of symmetrical or asymmetrical shape.

16. Process according to one of Claims 12 to 15, **characterized in that** the photochromic colouring agent is chosen from spirooxazines and derivatives thereof, spironaphthoxazines, naphthopyran and derivatives thereof, spiropyrans, nitrobenzylpyridines, spirolanes, titanium or zinc oxides doped with iron, aluminosilicates doped with metal ions or with a group chosen from Se, S, OH, SO₄²⁻ and WO₄²⁻.

17. Process according to one of Claims 12 to 16, **characterized in that** the second composition contains at least one additional monochromatic colouring agent.

18. Process according to one of Claims 12 to 17, **characterized in that** the monochromatic colouring agent is chosen from monochromatic dyes, monochromatic pigments and/or pearlescent agents.

19. Process according to Claim 17 or 18, **characterized in that** the monochromatic colouring agent is chosen from titanium oxide, zirconium oxide or cerium oxide and zinc oxide, iron oxide or chromium oxide, ferric blue, carbon black and barium, strontium, calcium or aluminium lakes, Sudan red, DC Red 17, DC Green 6, β-carotene, soybean oil, Sudan brown, DC Yellow 11, DC Violet 2, DC Orange 5, quinoline yellow, mica coated with titanium oxide, with iron oxide, with natural pigment or with bismuth oxychloride, such as coloured titanium mica.

20. Make-up kit comprising a product according to one of Claims 1 to 11.

21. Kit according to Claim 20, **characterized in that** it contains means for applying the first and second compositions to the skin and/or mucous membranes and/or superficial body growths.

22. Kit according to Claim 21, **characterized in that** the means are chosen from fine brushes, pens, brushes, feathers and pencils.

23. Kit according to one of Claims 20 to 22, **characterized in that** the first and second compositions are packaged in separate compartments or containers.

24. Cosmetic use of the product according to one of Claims 1 to 11, to make colour patterns appear or disappear on the skin and/or the lips and/or superficial body growths of human beings, depending on the presence or absence of ultraviolet light, by applying to the skin and/or the lips and/or superficial body growths a first coat of the first composition, and then applying, over a portion of the said first coat, a second coat of the second composition.

25. Made-up support comprising a first coat of a first composition comprising at least one photochromic colouring agent capable of producing at least one colour in the presence of ultraviolet light, and a second coat of a second composition partly applied onto the first coat and comprising at least one UV light screening agent.

26. Support according to Claim 25, **characterized in that** it is in the form of false nails, false eyelashes or wigs.

27. Support according to Claim 25 or 26, **characterized in that** the second coat is discontinuous.

28. Support according to one of Claims 25 to 27, **characterized in that** the second coat comprises patterns distributed in a random or ordered manner.

29. Support according to one of Claims 25 to 28, **characterized in that** the photochromic colouring agent is chosen from spirooxazines and derivatives thereof, spironaphthoxazines, naphthopyran and derivatives thereof, spiropyrans, nitrobenzylpyridines, spirolanes, titanium or zinc oxides doped with iron, aluminosilicates doped with metal ions or with a group chosen from Se, S, OH, SO₄²⁻ and WO₄²⁻.

30. Support according to one of Claims 25 to 29, **characterized in that** the UV light screening agent is chosen from treated or untreated titanium or zinc nanooxides, cinnamic derivatives, salicylic derivatives, camphor derivatives, sulphonic benzimidazole derivatives, triazine derivatives, benzophenone derivatives, dibenzoylmethane derivatives, β,β-diphenylacrylate derivatives, p-aminobenzoic acid derivatives, lipophilic screening polymers and screening silicones, and mixtures thereof.

31. Support according to one of Claims 25 to 30, **characterized in that** the second composition contains at least one monochromatic colouring agent.

## Patentansprüche

1. Kosmetisches Produkt zum Schminken, das eine erste und eine zweite Zusammensetzung umfaßt, die ein kosmetisch akzeptables Medium enthalten und getrennt voneinander konfektioniert sind, wobei die erste Zusammensetzung mindestens ein photochromes Farbmittel enthält, das befähigt ist, in Gegenwart von . UV-Strahlung mindestens eine Farbe hervorzurufen, und die zweite Zusammensetzung mindestens ein UV-Filter enthält.

2. Produkt nach Anspruch 1, **dadurch gekennzeichnet, daß** das photochrome Farbmittel ausgewählt ist unter Spirooxazinen und ihren Derivaten, Spironaphthoxazinen, Naphthopyran und seinen Derivaten, Spiropyranen, Nitrobenzylpyridinen, Spirolanen, Titanoxid oder Zinkoxid, die mit Eisen dotiert sind, und Aluminiumsilicaten, die mit Metallionen oder Gruppen, die unter Se, S, OH, SO₄²⁻ oder WO₄²⁻ ausgewählt sind, dotiert sind.

3. Produkt nach Anspruch 1 oder 2, **dadurch gekennzeichnet, daß** das UV-Filter unter den Nanooxiden von Titan oder Zink, die behandelt oder unbehandelt vorliegen, Zimtsäurederivaten, Salicylsäurederivaten, Campherderivaten, Sulfonsäurederivaten von Benzimidazol, Triazinderivaten, Benzophenonderivaten, Dibenzoylmethanderivaten, β,β-Diphenylacrylatderivaten, p-Aminobenzoesäurederivaten, lipophilen polymeren Filtern und lipophilen Siliconfiltern und deren Gemischen ausgewählt ist.

4. Produkt nach einem der vorhergehenden Ansprüche, **dadurch gekennzeichnet, daß** das UV-Filter in einer Menge von 0,01 bis 30 % und besser 0,5 bis 20 % des Gesamtgewichts der zweiten Zusammensetzung vorliegt.

5. Produkt nach einem der vorhergehenden Ansprüche, **dadurch gekennzeichnet, daß** die zweite Zusammensetzung mindestens ein einfarbiges Farbmittel enthält.

6. Produkt nach dem vorhergehenden Anspruch, **dadurch gekennzeichnet, daß** das einfarbige Farbmittel unter den einfarbigen Farbstoffen, einfarbigen Pigmenten und/oder Perlglanzpigmenten ausgewählt ist.

7. Produkt nach Anspruch 5 oder 6, **dadurch gekennzeichnet, daß** das einfarbige Farbmittel ausgewählt ist unter: Oxiden von Titan, Zirconium oder Cer sowie den Oxiden von Zink, Eisen oder Chrom, Eisenblau, Ruß, Lacken von Barium, Strontium, Calcium oder Aluminium, Sudanrot, DC Red 17, DC Green 6, β-Carotin, Sojaöl, Sudanbraun, DC Yellow 11, DC Violet 2, DC Orange 5, Chinolingelb, mit Titanoxid, Eisenoxid, natürlichem Pigment oder Bismutoxidchlorid überzogenem Glimmer, wie beispielsweise farbigem Titanglimmer.

8. Produkt nach einem der vorhergehenden Ansprüche, **dadurch gekennzeichnet, daß** jeder Farbstoff 0,01 bis 98 % und besser 0,05 bis 85 % des Gesamtgewichts der ersten bzw. der zweiten Zusammensetzung ausmacht.

9. Produkt nach einem der vorhergehenden Ansprüche, **dadurch gekennzeichnet, daß** es in Form von Make-up, Nagellack, Produkt zum Schminken des Körpers oder der Lippen, Wagenrouge oder Lidschatten konfektioniert ist.

10. Produkt nach einem der vorhergehenden Ansprüche, **dadurch gekennzeichnet, daß** jede der Zusammensetzungen als ölige oder wäßrige Lösung, öliges oder wäßriges Gel, Öl-in-Wasser-Emulsion oder Wasser-in-Öl-Emulsion, Dispersion von Öl in Wasser mit Hilfe von Vesikeln, wobei sich die Vesikel an der Grenzfläche Öl/Wasser befinden, oder Pulver vorliegt.

11. Produkt nach einem der vorhergehenden Ansprüche, **dadurch gekennzeichnet, daß** das kosmetisch akzeptable Medium außerdem mindestens einen Bestandteil enthält, der ausgewählt ist unter: Ölen, Lösungsmitteln, Wachsen, filmbildenden Polymeren, Füllstoffen, hydrophilen oder lipophilen Wirkstoffen, Verdickungsmitteln für die wäßrige Phase oder die Fettphase, grenzflächenaktiven Stoffen, Antioxidantien, Parfums, Weichmachern, Neutralisationsmitteln, Stabilisatoren und Konservierungsmitteln.

12. Verfahren zum Schminken der Haut, der Lippen und/oder der Hautanhangsgebilde des Menschen, das darin besteht, auf die Haut, die Lippen und/oder die Hautanhangsgebilde eine erste Schicht einer ersten Zusammensetzung aufzubringen, die ein kosmetisch akzeptables Medium und mindestens ein photochromes Farbmittel enthält, das in Gegenwart von UV-Strahlung mindestens eine Farbe hervorrufen kann, und dann auf einen Teil der ersten Schicht eine zweite Schicht einer zweiten Zusammensetzung aufzubringen, die ein kosmetisch akzeptables Medium und mindestens ein UV-Filter enthält.

13. Verfahren nach Anspruch 12, **dadurch gekennzeichnet, daß** die zweite Schicht diskontinuierlich ist.

14. Verfahren nach einem der Ansprüche 12 bis 13, **dadurch gekennzeichnet, daß** die zweite Schicht Motive umfaßt.

15. Verfahren nach einem der Ansprüche 12 bis 14, **dadurch gekennzeichnet, daß** die zweite Schicht Motive umfaßt, die zufällig oder geordnet, symmetrisch oder unsymmetrisch verteilt sind.

16. Verfahren nach einem der Ansprüche 12 bis 15, **dadurch gekennzeichnet, daß** das photochrome Farbmittel ausgewählt ist unter den Spirooxazinen und seinen Derivaten, Spironaphthoxazinen, Naphthopyran und seinen Derivaten, Spiropyranen, Nitrobenzylpyridinen, Spirolanen, Oxiden von Titan oder Zink, die mit Eisen dotiert sind, und Aluminiumsilicaten, die mit Metallionen oder einer Gruppe, die unter Se, S, OH, SO₄²⁻ oder WO₄²⁻ ausgewählt ist, dotiert ist.

17. Verfahren nach einem der Ansprüche 12 bis 16, **dadurch gekennzeichnet, daß** die zweite Zusammensetzung mindestens ein zusätzliches einfarbiges Farbmittel enthält.

18. Verfahren nach einem der Ansprüche 12 bis 17, **dadurch gekennzeichnet, daß** das einfarbige Farbmittel unter den einfarbigen Farbstoffen, einfarbigen Pigmenten und/oder Perlglanzpigmenten ausgewählt ist.

19. Verfahren nach Anspruch 17 oder 18, **dadurch gekennzeichnet, daß** das einfarbige Farbmittel ausgewählt ist unter: Oxiden von Titan, Zirconium oder Cer sowie den Oxiden von Zink, Eisen oder Chrom, Eisenblau, Ruß, Lacken von Barium, Strontium, Calcium oder Aluminium, Sudanrot, DC Red 17, DC Green 6, β-Carotin, Sojaöl, Sudanbraun, DC Yellow 11, DC Violet 2, DC Orange 5, Chinolingelb, mit Titanoxid, Eisenoxid, natürlichem Pigment oder Bismutoxidchlorid überzogenem Glimmer, wie beispielsweise farbigem Titanglimmer.

20. Kit zum Schminken, der ein Produkt nach einem der Ansprüche 1 bis 11 enthält.

21. Kit nach Anspruch 20, **dadurch gekennzeichnet, daß** er Mittel zum Aufbringen der ersten und zweiten Zusammensetzung auf die Haut und/oder die Schleimhäute und/oder die Hautanhangsgebilde umfaßt.

22. Kit nach Anspruch 21, **dadurch gekennzeichnet, daß** die Mittel unter den Pinseln, Füllern, Bürsten, Federn und Stiften ausgewählt sind.

23. Kit nach einem der Ansprüche 20 bis 22, **dadurch gekennzeichnet, daß** die erste Zusammensetzung und zweite Zusammensetzung in unterschiedlichen Abteilungen oder Behältern konfektioniert sind.

24. Kosmetische Verwendung eines Produkts nach einem der Ansprüche 1 bis 11, um auf der Haut und/oder den Lippen und/oder den Hautanhangsgebilden des Menschen in Abhängigkeit vom Vorhandensein oder Fehlen von UV-Strahlung farbige Motive erscheinen oder verschwinden zu lassen, indem auf die Haut, die Lippen und/oder die Hautanhangsgebilde eine erste Schicht der ersten Zusammensetzung und anschließend auf einen Teil der ersten Schicht eine zweite Schicht der zweiten Zusammensetzung aufgebracht wird.

25. Geschminkter Träger, der eine erste Schicht einer ersten Zusammensetzung, die mindestens ein photochromes Farbmittel enthält, das in Gegenwart von UV-Strahlung mindestens eine Farbe hervorrufen kann, und eine zweite Schicht einer zweiten Zusammensetzung umfaßt, die auf einem Teil der ersten Schicht abgeschieden wurde und mindestens ein UV-Filter enthält.

26. Träger nach Anspruch 25, **dadurch gekennzeichnet, daß** er in Form von künstlichen Nägeln, falschen Wimpern und Perücken vorliegt.

27. Träger nach Anspruch 25 oder 26, **dadurch gekennzeichnet, daß** die zweite Schicht diskontinuierlich ist.

28. Träger nach einem der Ansprüche 25 bis 27, **dadurch gekennzeichnet, daß** die zweite Schicht Motive umfaßt, die zufällig oder geordnet verteilt sind.

29. Träger nach einem der Ansprüche 25 bis 28, **dadurch gekennzeichnet, daß** das photochrome Farbmittel ausgewählt ist unter den Spirooxazinen und deren Derivaten, Spironaphthoxazinen, Naphthopyran und seinen Derivaten, Spiropyranen, Nitrobenzylpyridinen, Spirolanen, Titanoxid und Zinkoxid, die mit Eisen dotiert sind, Aluminiumsilicaten, die mit Metallionen oder mit einer Gruppe dotiert sind, die unter Se, S, OH, SO₄²⁻ oder WO₄²⁻ ausgewählt ist.

30. Träger nach einem der Ansprüche 25 bis 29, **dadurch gekennzeichnet, daß** das UV-Filter unter den Nanooxiden von Titan oder Zink, die behandelt oder unbehandelt vorliegen, Zimtsäurederivaten, Salicylsäurederivaten, Campherderivaten, Sulfonsäurederivaten von Benzimidazol, Triazinderivaten, Benzophenonderivaten, Dibenzoylmethanderivaten, β,β-Diphenylacrylatderivaten, p-Aminobenzoesäurederivaten, lipophilen polymeren Filtern und lipophilen Siliconfiltern und deren Gemischen ausgewählt ist.

31. Träger nach einem der Ansprüche 25 bis 30, **dadurch gekennzeichnet, daß** die zweite Zusammensetzung mindestens ein einfarbiges Farbmittel enthält.
